# DEMANDE DE BREVET EUROPEEN

(11) **EP 0 666 476 A1**
(43) Date de publication de la demande: **09.08.1995**
(21) Numéro de dépôt: 95400072.5
(22) Date de dépôt: 13.01.1995
(51) Int. Cl.: G01N 33/561, G01N 27/447

(54) **Dispositif pour l'immunofixation sur un support (plaque) unique, d'échantillons différents**

(30) Priorité: 14.01.1994 FR 9400393
(71) Demandeur: SEBIA, F-92130 Issy-Les-Moulineaux (FR)
(72) Inventeur: Barouh, Guy, F-92130 Issy-les-Moulineaux (FR)
(74) Mandataire: Desaix, Anne

(57) **Abrégé**

L'invention concerne une plaque d'électrophorèse pour la réalisation d'une immunofixation, caractérisée en ce qu'elle comporte un nombre de pistes d'électrophorèse suffisant pour permettre la migration électrophorétique puis la caractérisation par immunofixation des constituants protéiques de plusieurs échantillons biologiques distincts, les pistes d'électrophorèse de la zone d'incubation de chaque échantillon ayant une largeur suffisante pour visualiser les constituants après migration, notamment comprise entre environ 2 mm et environ 5 mm et la largeur de l'espace entre deux pistes (espace inter-pistes) étant déterminée de telle sorte que les antisérums ne diffusent pas d'une piste vers une autre, cet espace ayant notamment une largeur comprise entre 2 mm et environ 3 mm.

L'invention concerne aussi un masque permettant le dépôt localisé de réactifs au niveau des pistes d'électrophorèse en vue de réaliser une immunofixation, caractérisé en ce qu'il comprend un nombre déterminé d'ouvertures, de préférence égal au nombre de pistes d'électrophorèse, lesdites ouvertures ayant une largeur comprise entre environ 2 mm et environ 5 mm et étant séparées les unes des autres par un espace d'environ 2 mm à environ 3 mm.

## Description

L'invention concerne un nouveau dispositif permettant l'immunofixation simultanée sur un support (de type plaque d'électrophorèse) unique, de composés présents dans des échantillons biologiques différents.

La technique d'immunofixation qui associe une électrophorèse à la formation de précipités sur gel est connue depuis longtemps. Cette technique a notamment été décrite par Alper CA et Johnson AM Vox. Sang. 17:445 (1969), Cawley LP et al Clin. Chem. 22:1262 (1976), Ritchie RF et Smith R Clin. Chem. 22:497,1735,1982 (1976). Cette technique est d'usage courant en laboratoire d'analyse clinique et permet l'identification d'anomalies dans différents liquides biologiques, par exemple dans du sérum, de l'urine ou du liquide céphalo-rachidien.

Cette technique comporte principalement les étapes suivantes :
1) séparation des constituants protéiques du sérum ou du liquide testé par électrophorèse sur une plaque notamment sur gel, par exemple sur gel d'agarose ;
2) réaction immunologique avec des anticorps spécifiques des protéines séparées ;
3) révélation des complexes immunologiques formés.

Les conditions pour la réalisation de ces étapes ont été décrites dans l'art antérieur.

Les dispositifs utilisés comportent en outre la possibilité de réaliser sur la même plaque, notamment le même gel, un témoin obtenu par fixation de l'ensemble des antigènes présents dans l'échantillon à l'aide par exemple d'un antisérum non spécifique, ou d'un fixateur de protéines.

De façon générale pour la mise en oeuvre d'une immunofixation, l'anticorps ou les anticorps utilisés pour révéler les antigènes séparés doit (doivent) se trouver en léger excès ou proche de l'équivalence en titre et en quantité, par rapport à l'antigène présent sur le gel. Dans ces conditions, les complexes antigènes-anticorps sont insolubles et restent emprisonnés dans les mailles du gel.

Les protéines non précipitées lors de la réaction immunologique sont éliminées du gel par lavage (pompage avec papier filtre et lavage en eau physiologique). Le gel est alors séché et coloré avec un colorant spécifique des protéines permettant ainsi la mise en évidence des protéines précipitées. Un colorant couramment utilisé dans cette technique est le noir amide.

Une électrophorèse réalisée conjointement sur le même support, sur un échantillon témoin permet une comparaison directe entre l'anomalie rencontrée sur le profil protéique à l'aide des anticorps spécifiques et des bandes révélées au niveau du témoin.

Plusieurs dispositifs sont actuellement disponibles dans le commerce pour réaliser à partir d'un échantillon, la détection de protéines par immunofixation.

On citera à titre d'exemple le dispositif vendu sous la dénomination HYDRAGEL® IF par la société SEBIA (Issy les Moulineaux, France). Conformément à ce dispositif, on utilise un gel d'électrophorèse sur lequel on dépose au niveau d'un nombre de pistes déterminé, une quantité donnée d'un échantillon qui est ensuite soumis à une électrophorèse. Un masque comportant un nombre d'ouvertures égal au nombre de pistes du gel, et coïncidant avec elles, est ensuite déposé sur le gel.

Après le dépôt du masque, un ou plusieurs antisérums spécifiques et/ou non-spécifiques, et/ou le cas échéant un fixateur de protéines sont déposés au niveau des pistes déterminées par la présence du masque sur le gel de façon à permettre la réaction des antigènes présents dans l'échantillon et des anticorps contenus dans les antisérums. Les complexes immunologiques sont ensuite mis en évidence selon les techniques classiques.

Selon le dispositif HYDRAGEL® IF, le gel utilisé pour la réalisation d'une immunofixation dite "simple" (IF simple), c'est à dire permettant la recherche d'antigènes dans un seul échantillon dont on recherche la composition, a les dimensions suivantes 83 mm de largeur sur 101 mm de longueur. Ce gel comporte 6 pistes d'électrophorèse permettant la réalisation d'un témoin et l'immunofixation avec des immunsérums spécifiques de type anti-IgG, anti-IgA, anti-IgM, anti-Kappa et anti-Lambda.

Pour la réalisation de cette immunofixation dite simple, les quantités d'échantillon et d'antisérum déposées par unité de surface sont sensiblement identiques et de l'ordre de 70 µl ou 80 µl pour chacun.

La présente invention a pour objet de proposer la réalisation d'une immunofixation sur plusieurs échantillons distincts, mais sur une unique plaque d'électrophorèse.

On a déjà proposé dans l'état antérieur de la technique, la réalisation de tests d'immunofixation simultanément à partir de deux échantillons différents. Les dispositifs connus à cet égard sont caractérisés par l'utilisation de plaques d'électrophorèse dont la taille est augmentée voire doublée par rapport à la taille des plaques notamment des gels couramment employés pour la réalisation d'immunofixations dites simples.

De tels dispositifs nécessitent le plus souvent la mise en oeuvre de matériel spécialement adapté s'agissant des dimensions, par exemple de cuves d'électrophorèse, et de boîtes de lavage et d'incubation, différentes du matériel utilisé pour la réalisation d'immunofixations dites simples.

Les inventeurs de la présente demande ont mis en évidence qu'il est possible d'effectuer simultanément sur une seule plaque d'électrophorèse, des immunofixations en vue de rechercher la composition de plusieurs échantillons distincts, et notamment de réaliser des immunofixations doubles (sur deux échantillons) en utilisant des plaques de mêmes dimensions que celles des plaques utilisées en immunofixation simple, sans altérer la sensibilité ni la praticabilité du test.

La technique utilisée pour réaliser sur une même plaque, une détection par immunofixation à partir de plusieurs, de préférence deux, échantillons distincts, a consisté à augmenter le nombre de pistes d'électrophorèse, en particulier à le doubler tout en conservant l'ordre de grandeur des dimensions d'ensemble du support d'électrophorèse habituellement utilisé pour l'immunofixation simple, sans que la qualité du test d'immunofixation soit altérée.

En particulier les inventeurs ont défini des conditions qui permettent de diminuer la dimension des pistes d'électrophorèse et de les rapprocher sur la plaque sans néanmoins observer par exemple des phénomènes de diffusion et de mélange d'une piste à l'autre, des antisérums appliqués pour effectuer l'immunofixation même dans le cas d'incubation prolongée (jusqu'à 30 mn), phénomènes qui nuiraient à la qualité du test. De même la réduction de la dimension, au moins de la largeur des pistes de la plaque d'électrophorèse et de l'espace entre les pistes (espace inter-pistes) ne diminue pas la qualité du test en ce sens que la visualisation et la lecture des résultats reste satisfaisante.

Le dispositif de l'invention peut être réalisé à partir des plaques habituellement utilisées pour effectuer des immunofixations en particulier à l'aide de gels d'électrophorèse tels que les gels d'agarose (à 1 %, 2 %, ...).

De même le masque utilisé pour déposer de façon localisée les antisérums sur la plaque d'électrophorèse après la migration électrophorétique est préparé à partir de matériaux couramment employés dans le domaine. Les échantillons biologiques testés sont des liquides biologiques, par exemple du sérum, de l'urine ou du liquide céphalo-rachidien.

En outre, de façon tout à fait intéressante, les inventeurs ont constaté que les conditions définies pour la réalisation de l'invention permettaient de diminuer la quantité d'échantillon déposée et ce malgré l'accroissement du nombre de pistes d'électrophorèse.

L'invention a pour objet une plaque d'électrophorèse pour la réalisation d'immunofixation telle que les dessins des pistes d'électrophorèse individualisées sont soit reproduits sur le support du gel, soit regroupés dans un cadre unique correspondant aux dimensions de l'ensemble des pistes nécessaires à l'analyse.

L'invention a donc pour objet une plaque d'électrophorèse pour la réalisation d'une immunofixation, qui selon un premier mode de réalisation de l'invention, est caractérisée en ce qu'elle comporte un nombre de pistes d'électrophorèse suffisant pour permettre la migration électrophorétique puis la caractérisation par immunofixation des constituants protéiques de plusieurs échantillons biologiques, les pistes d'électrophorèse de la zone d'incubation de chaque échantillon ayant une largeur suffisante pour visualiser les constituants après migration, notamment une largeur comprise entre environ 2 mm et environ 5 mm et, la largeur de l'espace entre deux pistes (espace inter-pistes) étant déterminée de telle sorte que les antisérums ne diffusent pas d'une piste vers une autre, cet espace ayant notamment une largeur comprise entre 2 mm et environ 3 mm.

Une telle plaque permet la réalisation simultanée de plusieurs immunofixations, en particulier de deux immunofixations sans que les dimensions de la plaque constituée de préférence par un gel, soient modifiées par rapport aux dimensions des gels habituellement utilisés pour effectuer des immunofixations simples. Le dispositif de l'invention présente l'avantage de réduire la quantité des réactifs utilisés pour procéder à l'immunofixation.

Selon une première variante de réalisation de la plaque de l'invention, la largeur des pistes d'électrophorèse est d'environ 4,75 mm.

Selon une autre variante de réalisation préférée de l'invention, l'espace inter-pistes a une largeur d'environ 2,55 mm.

Les dimensions globales de la plaque d'électrophorèse sont avantageusement comprises entre 75 mm et 90 mm pour la largeur, et comprises entre 95 mm et 110 mm pour la longueur.

Une plaque d'électrophorèse particulièrement avantageuse pour la réalisation d'une immunofixation selon l'invention est caractérisé en ce qu'il comporte 12 pistes, et en ce que ses dimensions sont les suivantes :
- longueur de la plaque : environ 102 mm
- largeur de la plaque : environ 82 mm
- largeur de chaque piste : environ 4,75 mm
- espace inter-pistes : environ 2,55 mm.

De préférence, la hauteur des pistes d'électrophorèse est d'environ 40 mm et l'espace entre deux groupes de 6 pistes d'électrophorèse est de 3 mm environ.

L'invention a également pour objet un masque permettant le dépôt localisé de réactifs au niveau des pistes d'électrophorèse de la plaque décrite ci-dessus, en vue de réaliser une immunofixation, caractérisé en ce qu'il comprend un nombre déterminé d'ouvertures, de préférence égal au nombre de pistes d'électrophorèse, lesdites ouvertures ayant une largeur comprise entre environ 2 mm et environ 5 mm et étant séparées les unes des autres par un espace d'environ 2 mm à environ 3 mm.

Un masque particulièrement préféré est réalisé de telle façon que la largeur des ouvertures qu'il comporte est d'environ 4,75 mm.

Selon un autre mode de réalisation préféré de l'invention, le masque est tel que l'espace entre les ouvertures qu'il comporte a une largeur d'environ 2,55 mm.

Selon un autre mode de réalisation de l'invention, les pistes d'électrophorèse de la zone d'incubation pour l'immunofixation des échantillons après migration ne sont pas imprimées individuellement sur le support ou sur le gel, mais correspondent exactement aux ouvertures du masque déposé après la migration des échantillons sur le gel. Dans ce cas, les pistes apparaissent comme étant regroupées dans un cadre unique sur la plaque d'électrophorèse, ledit cadre ayant les dimensions indiquées ci-dessus pour la plaque d'électrophorèse.

L'invention vise également un ensemble permettant la réalisation d'immunofixations multiples, par exemple d'une immunofixation double, caractérisé en ce qu'il comporte une plaque répondant à l'un des modes de réalisation décrits ci-dessus et un masque tel que défini précédemment.

Un procédé pour la réalisation d'immunofixations multiples selon l'invention comprend les étapes suivantes :
- le dépôt d'une quantité comprise entre 1 µl et 8 µl, de préférence d'environ 5 µl, de chaque échantillon à analyser à une dilution allant de 1/10 à 1/50, sur chaque piste d'une plaque d'électrophorèse décrite ci-dessus,
- la migration électrophorétique des échantillons de façon à séparer les constituants protéiques qu'il contiennent,
- l'application d'un masque décrit ci-dessus sur le support d'électrophorèse,
- le dépôt à travers chaque ouverture du masque d'une quantité comprise entre 30 et 40 µl environ de préférence environ 35 µl, de réactifs de fixation spécifiques et/ou non spécifiques des protéines, notamment des antisérums spécifiques,
- l'incubation des réactifs de façon à permettre leur réaction avec les constituants protéiques des échantillons, en particulier la formation de complexes immunologiques,
- la détection des complexes immunologiques formés.

La quantité de réactif déposée sur la plaque est d'environ 15 à environ 25 µl/cm² de piste de préférence environ 18,5 µl/cm².

Selon une variante particulière de la réalisation du procédé, le masque peut être retiré dès que les réactifs ont pénétré totalement dans la plaque avant la fin de la période d'incubation des réactifs.

Les techniques et matériaux mis en oeuvre pour la réalisation de l'invention sont ceux habituellement utilisés pour effectuer des immunofixations.

Cependant, le dispositif de l'invention permet avantageusement d'utiliser une quantité moindre d'échantillons et de réactifs et d'effectuer sur une même plaque d'électrophorèse plusieurs analyses simultanées.

De façon tout à fait intéressante, la sensibilité de la détection des constituants de l'échantillon et en particulier des paraprotéines susceptibles de se trouver dans les échantillons testés est conservée par rapport à la sensibilité de détection habituellement obtenue avec une immunofixation de type simple.

S'agissant des matériaux employés pour réaliser le masque de l'invention, on pourra citer des plastiques souples, par exemple des polyesters, le terphane, d'épaisseur d'environ 50 à 75 µm.

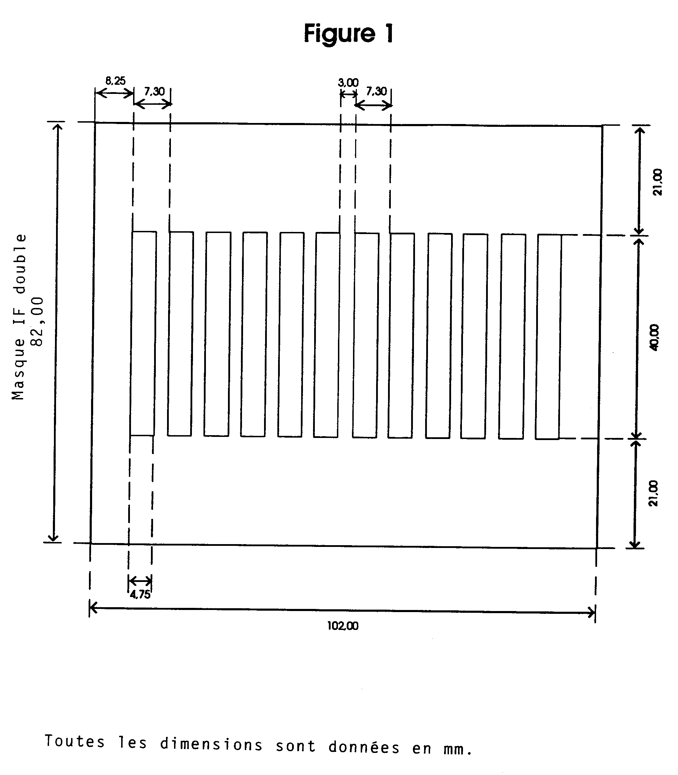

La figure 1 représente un masque comportant 12 pistes, de format égal à 82 mm sur 102 mm, réalisé en terphane, d'épaisseur 75 µm.

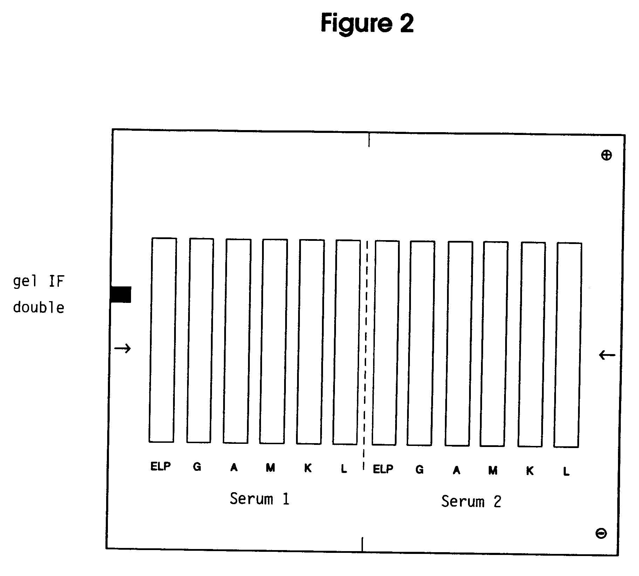

La figure 2 représente un gel permettant la réalisation d'une immunofixation double et comportant 12 pistes pour l'analyse de deux sérums différents.

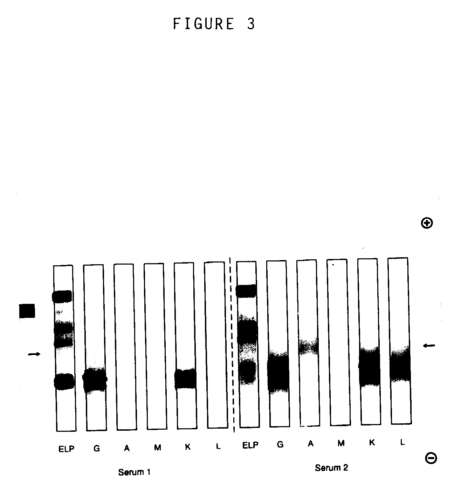

La figure 3 représente les résultats d'une immunofixation double.

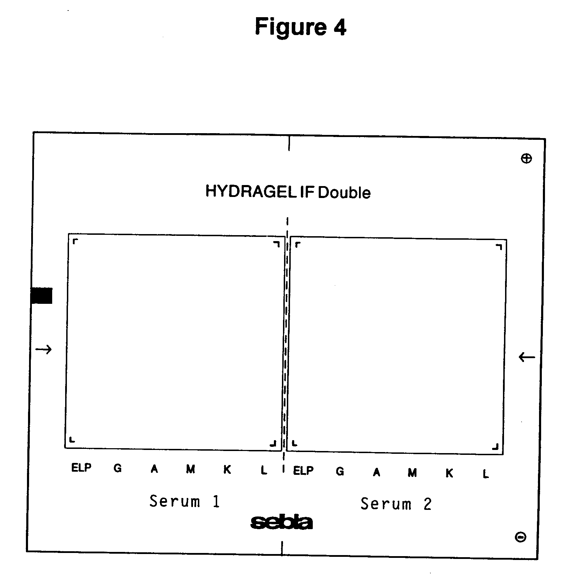

La figure 4 représente une plaque d'électrophorèse sur laquelle les pistes sont regroupées au sein d'un cadre unique.

## Revendications

1. Plaque d'électrophorèse pour la réalisation d'une immunofixation, caractérisée en ce qu'elle comporte un nombre de pistes d'électrophorèse suffisant pour permettre la migration électrophorétique puis la caractérisation par immunofixation des constituants protéiques de plusieurs échantillons biologiques distincts, les pistes d'électrophorèse de la zone d'incubation de chaque échantillon ayant une largeur suffisante pour visualiser les constituants après migration, notamment comprise entre environ 2 mm et environ 5 mm et la largeur de l'espace entre deux pistes (espace inter-pistes) étant déterminée de telle sorte que les antisérums ne diffusent pas d'une piste vers une autre, cet espace ayant notamment une largeur comprise entre 2 mm et environ 3 mm.

2. Plaque d'électrophorèse pour la réalisation d'une immunofixation selon la revendication 1, caractérisée en ce que la largeur des pistes est d'environ 4,75 mm.

3. Plaque d'électrophorèse pour la réalisation d'une immunofixation selon la revendication 1 ou la revendication 2, caractérisée en ce que l'espace inter-pistes a une largeur d'environ 2,55 mm.

4. Plaque d'électrophorèse pour la réalisation d'une immunofixation selon l'une quelconque des revendications 1 à 3, caractérisée en ce que sa largeur est comprise entre environ 75 mm et 90 mm et sa longueur est comprise entre environ 95 mm et 110 mm.

5. Plaque d'électrophorèse pour la réalisation d'une immunofixation selon l'une quelconque des revendications 1 à 4, caractérisée en ce qu'elle comporte 12 pistes et en ce que ses dimensions sont les suivantes :
- longueur de la plaque : 102 mm
- largeur de la plaque : 82 mm
- largeur de chaque piste : 4,75 mm
- espace inter-pistes : 2,55 mm.

6. Plaque d'électrophorèse pour la réalisation d'une immunofixation selon la revendication 5, caractérisée en ce que la hauteur de piste est d'environ 40 mm et l'espace entre 2 groupes de 6 pistes est de 3 mm.

7. Masque permettant le dépôt localisé de réactifs au niveau des pistes d'électrophorèse selon l'une quelconque des revendications 1 à 6 en vue de réaliser une immunofixation, caractérisé en ce qu'il comprend un nombre déterminé d'ouvertures, de préférence égal au nombre de pistes d'électrophorèse, lesdites ouvertures ayant une largeur comprise entre environ 2 mm et environ 5 mm et étant séparées les unes des autres par un espace d'environ 2 mm à environ 3 mm.

8. Masque selon la revendication 7, caractérisé en ce que la largeur des ouvertures est d'environ 4,75mm.

9. Masque selon la revendication 7 ou la revendication 8, caractérisé en ce que l'espace entre les ouvertures a une largeur d'environ 2,55 mm.

10. Ensemble permettant la réalisation d'immunofixations multiples, par exemple double, caractérisé en ce qu'il comporte une plaque selon l'une quelconque des revendications 1 à 5 et un masque selon l'une quelconque des revendications 6 à 9.

11. Ensemble permettant la réalisation d'immunofixations multiples, par exemple double, caractérisé en ce qu'il comporte une plaque d'électrophorèse regroupant des pistes d'électrophorèse non imprimées individuellement et comprise dans un cadre unique, et un masque selon l'une quelconque des revendications 6 à 9.

12. Procédé pour la réalisation d'immunofixations multiples comprenant les étapes de :
- le dépôt d'une quantité comprise entre 1 µl et 8 µl, de préférence d'environ 5 µl, de chaque échantillon à analyser à une dilution allant de 1/10 à 1/50, sur chaque piste d'une plaque d'électrophorèse selon l'une quelconque des revendications 1 à 5,
- la migration électrophorétique des échantillons de façon à séparer les constituants protéiques qu'il contiennent,
- l'application d'un masque selon l'une quelconque des revendications 6 à 9, sur le support d'électrophorèse
- le dépôt à travers chaque ouverture du masque d'une quantité comprise entre 30 µl et 40 µl environ de préférence environ 35 µl, de réactifs de fixation spécifiques et/ou non spécifiques des protéines, notamment des antisérums spécifiques
- l'incubation des réactifs de façon à permettre leur réaction avec les constituants protéiques des échantillons, en particulier la formation de complexes immunologiques,
- la détection des complexes immunologiques formés.
